# EUROPÄISCHE PATENTSCHRIFT

(11) **EP 1 851 542 B1**
(45) Veröffentlichungstag und Bekanntmachung des Hinweises auf die Patenterteilung: **14.04.2010**
(21) Anmeldenummer: 06706814.8
(22) Anmeldetag: 10.02.2006
(51) Int. Cl.: G01N 33/50

(54) **VERWENDUNG VON THIOPEPTOLIDEN ZUR AKTIVITÄTSBESTIMMUNG VON ADAM-TS PROTEASEN**
USE OF THIOPEPTOLIDES FOR DETERMINING THE ACTIVITY OF ADAMTS PROTEASES
UTILISATION DE THIOPEPTOLIDES POUR DETECTER L'ACTIVITE DE PROTEASES ADAM-TS

(43) Veröffentlichungstag der Anmeldung: 07.11.2007
(73) Patentinhaber: Sanofi-Aventis, 75013 Paris (FR)
(72) Erfinder: WEITHMANN, Klaus-Ulrich, 65719 Hofheim (DE); JESKE, Volker, 61479 Glashütten (DE)
(74) Vertreter: Schneider, Rudolf
(86) Internationale Anmeldenummer: PCT/EP2006/001184
(87) Internationale Veröffentlichungsnummer: WO 2006/092203

(56) Entgegenhaltungen:
- US-A- 4 569 907
- US-A1- 2002 142 362
- SINGH S ET AL: "Design of a novel fluorogenic peptide substrate for aggrecanase activity" JOURNAL OF PEPTIDE SCIENCE, Bd. 8, Nr. Supplement, 2002, Seite S210, XP008063421 & 27TH EUROPEAN PEPTIDE SYMPOSIUM; SORRENTO, ITALY; AUGUST 31-SEPTEMBER 06, 2002 ISSN: 1075-2617
- WESTLING JENNIFER ET AL: "Examination of the substrate specificity of ADAMTS-1 using a novel peptide-based assay" MOLECULAR BIOLOGY OF THE CELL, Bd. 11, Nr. Supplement, Dezember 2000 (2000-12), Seite 482a, XP008063458 & 40TH AMERICAN SOCIETY FOR CELL BIOLOGY ANNUAL MEETING; SAN FRANCISCO, CA, USA; DECEMBER 09-13, 2000 ISSN: 1059-1524
- RODRIGUEZ-MANZANEQUE JUAN CARLOS ET AL: "ADAMTS1 cleaves aggrecan at multiple sites and is differentially inhibited by metalloproteinase inhibitors" BIOCHEMICAL AND BIOPHYSICAL RESEARCH COMMUNICATIONS, Bd. 293, Nr. 1, 26. April 2002 (2002-04-26), Seiten 501-508, XP002378316 ISSN: 0006-291X
- KOKAME KOICHI ET AL: "VWF73, a region from D1596 to R1668 of von Willebrand factor, provides a minimal substrate for ADAMTS-13." BLOOD, Bd. 103, Nr. 2, 15. Januar 2004 (2004-01-15), Seiten 607-612, XP002378317 ISSN: 0006-4971

## Beschreibung

Die vorliegende Erfindung betrifft die Verwendung eines Thiopeptolids der Formel Ac-Pro-Leu-Gly-[2-mercapto-4-methyl-pentanoyl]-Leu-Gly-OC₂H₅, worin Ac eine Acetylgruppe bedeutet, oder ein Salz davon, als ein Substrat für die ADAM-TS-1 Protease oder ADAM-TS-4 Protease und ein Verfahren zum Auffinden eines ADAM-TS-1 oder eines ADAM-TS-4 Proteasemodulators.

Eine intakte Gelenkknorpelmatrix ist die entscheidende Voraussetzung für die Funktion aller Gelenke des tierischen und menschlichen Körpers. Schädigung des Gelenkknorpels führt zu arthritischen Krankheiten, wie Osteoarthrose (Osteoarthritis) und Rheuma, die durch Dysfunktion und schließlich Bewegungsunfählgkeit des betroffenen Tieres oder Menschen gekennzeichnet sind.

Zu den weiteren Krankheiten, die durch einen gestörten Matrixabbau gekennzeichnet sind, müssen auch die vielfältigen Formen der Krebserkrankungen gezählt werden, insbesondere die Metastasierung von Tumoren.

Es ist schon länger bekannt, dass Matrix MetalloProteasen (MMPs) am Abbau des Aggrecans und Collagens im Knorpel beteiligt sind. Hierzu zählt zum Beispiel die Gruppe der Matrixine, die alle bekannten MMPs von MMP-1, 2 usw. bis MMP-16 umfasst. Eine weitere Gruppe, nämlich die Proteasen der ADAM-TS-Familie (Nagase, H. et al. (2003), Arthritis Research Therapy, 5, 94 - 103), spielt ebenfalls eine entscheidende Rolle beim Abbau von Gewebematrix, wodurch es zu einer Schädigung der Knorpelmatrix kommt. Die Aktivität dieser Proteasen, insbesondere ADAM-TS 1, ADAM-TS 4 und ADAM-TS 5, die auch als ,Aggrecanasen' bezeichnet werden, ist die Ursache für Krankheiten, die durch einen gestörten Matrixabbau gekennzeichnet sind, wie Osteoarthrose, Rheuma und Krebs. ADAM-TS kann insbesondere Proteoglycan, aber auch andere Matrixbestandtelle, wie Hyaluronan oder Collagen, spalten. Weitere Wirkungen von ADAM-TS 1, 4, 5 und 11, aber auch ADAM-TS 13, sind entscheidend bei Entzündungsprozessen, Angiogenese, Zellmigration und Blutgerinnung, bzw. Blutcoagulation (Apte, S. S. (2004), The International Journal of Biochemistry & Cell Biology, 36, 981-985). So wurde z. B. gefunden, dass ADAM-TS 1 Aggrecan an verschiedenen Stellen spaltet und durch Metallproteinase-Inhibitoren unterschiedlich gehemmt wird.

Es ist daher eine wichtige Aufgabe der Arzneimitteiforschung, dass einerseits die enzymatische Aktivität der an den Krankheiten beteiligten Proteasen im gefährdeten oder bereits erkrankten Gewebe, z. B. Knorpelgewebe, oder Blut nachgewiesen werden kann. Andererseits ist es aber auch von besonderer Bedeutung, Arzneimittel zu entwickeln, die in der Lage sind, einzelne, mehrere oder alle relevanten Proteasen zu inhibieren.

Die enzymatische (proteolytische) Aktivität der beteiligten Proteasen kann in vitro gemessen werden, indem die betreffende Protease mit den entsprechenden hochmolekularen Matrixkomponenten, z.B. Proteoglycan oder Collagen, inkubiert wird und die Entstehung der Abbauprodukte gemessen wird.

Für die Isolierung und Quantifizierung der heterogenen Abbauprodukte, also z. B. Collagenbruchstücke und Proteinfragmente, stehen dem Fachmann verschiedene Verfahren zur Verfügung, die regelmäßig aufwändige Prozeduren, wie Antikörpererkennung von spezifischen Schnittstellen bzw. massenspektrometrische Untersuchungen erforderlich machen.

Es ist z. B. bereits beschrieben worden, dass zur Bestimmung der Aktivität von ADAM-TS 4 ein recombinantes Substrat verwendet werden kann, das wichtige Strukturelemente der interglobulären Domäne des natürlichen Aggrecans enthält. Das recombinante Aggrecan vom Molekulargewicht 72 kDa wird in COS-Zellen exprimiert. Die Bestimmung der Aggrecanaseaktivität erfordert neben dem Einsatz des hochmolekularen Aggrecan-Moleküls weitere aufwändige Schritte, wie Strukturelemente, die die Signalsequenz von CD5, das FLAG-epitop für die M1 monoclonale Antikörper-Bestimmung, die Hinge-Region des humanen IgGI, cDNA für das erwähnte recombinante Substrat, einschließlich deren Vectoren, wie dies im Detail in EP 0785 274 und auch in Hörber, Chr. et al. (2000) Matrix Biology, 19, 533-543 beschrieben ist. Westling, J. et al. (2000), Mol. Biol. Cell 11, 482a beschreiben einen quantitativen Fluoreszenzpeptid-basierenden Test zur Analyse einer Reihe von Peptiden mit Aminosäureaustauschen.

Auch die Verwendung kürzerer Fragmente des Aggrecan-Moleküls ist bereits bekannt. In WO 00105256 ist beschrieben worden, dass diese Peptidfragmente aus 20 bis 40

Aminosäurebausteine bestehen können. Allerdings ist es nicht möglich, Peptide, die weniger als 20 Aminosäuren enthalten, mit Aggrecanase umzusetzen, was besonders nachteilig ist. Dies konnte bestätigt werden (siehe experimenteller Teil).

Für die Bestimmung der Proteasen der Matrixin-Familie werden gemäß dem Stand der Technik zur Bestimmung der enzymatischen (proteolyiischen) Aktivität niedermolekulare, synthetisch einfach zugängliche Moleküle als Substrate der Proteasen gespalten, wobei auf Grund der besonderen Beschaffenheit dieser Substrate durch die Spaltung z. B. ein optisches Signal in der Regel im sichtbaren oder ultravioletten Weilenlängenbereich freigesetzt wird, das quantifiziert werden kann.

Ein bekanntes Beispiel ist das von Knight, C. G. et al. (1992) FEBS, 296, 263-266 beschriebene (7-meihoxycoumarin-4-yl)acetyl-Pro-Leu-Gly-Leu-3-(2',4'-dinitro-phenyl)-L-2,3-diaminopropionyl-Ala-Arg-NH2 (Bachern, Heidelberg, Deutschland), das, durch bestimmte Matrixmetalloproteinasen gespalten wird, und dabei ein messbares fluorimetrisches Signal freisetzt, das zur Berechnung der Enzymaktivität herangezogen wird. So konnte bestätigt werden, dass es möglich ist, (7-methoxycoumarin-4-yl)acetyl-Pro-Leu-Gly-Leu-3-(2',4'-dinitrophenyl)-L-2,3-diamino-propionyl-Ala-Arg-NH2 mit den Proteasen MMP-3 und MMP-8 umzusetzen (siehe experimenteller Teil). Auch zahlreiche weitere derartige Substrate konnten von MMP-3 bzw. MMP-8 umgesetzt werden.

Es ist auch schon bekannt, dass gewisse Substrate aus der Stoffklasse der Thiopeptolide von Collagenase bzw. membranständigen Matrixmetalloproteinasen umgesetzt werden, vgl. EP 0149593 sowie US 200210142362. Ein auf 16 Aminosäuren N-terminal zur Aggrecanase-Schnittstelle verkürztes Peptid wird von Aggrecanase jedoch nicht mehr gespalten. Singh, W. et al. (2002) J. Peptide Sc. 8, S210 beschreiben ein weiteres Aggrecanase-Peptidsubstrat der Formel NH2-Leu-Pro-Arg-Dpa-le-Thr-Glu-Gly-Glu-Ala-Arg-Gly-Lys(MCA)-NH und. Kokame, K. et al. (2004) Blood, 1,607-612 beschreiben eine aus 73 Aminosäuren bestehende Region des von Willebrandfaktors, die ein Minimalsubstrat von ADAMTS-13 darstellt.

Auch in eigenen Experimenten konnte bestätigt werden, dass kürzerkettige Peptide, selbst wenn sie die Sequenz Glu-Ala enthielten, von ADAM-TS nicht gespalten werden konnten. Hingegen ist es bereits bekannt, dass die Mitglieder anderer Protease-Familien, z. B. die Matrixine oder die Cathepsine, Oligopeptide spalten können.

Die Verwendung von Oligopeptiden in derartigen Experimenten ist deshalb besonders erwünscht, weil sie einer chemischen Synthese leicht zugänglich sind. Ein weiterer Vorteil der Oligopeptide ist, dass einzelne Peptidbausteine chemisch verändert werden können, z; B. auch mit solchen chromophoren Gruppen verknüpft werden können, die es erlauben, die Spaltung des Peptides mittelbar oder unmittelbar spektrometrisch, z. B. kolorimetrisch zu verfolgen. In entsprechender Weise kann auch die Wirkung von Enzyminhibitoren oder -aktivatoren leicht bestimmt werden, indem man die proteolflische Aktivität der betreffenden Protease nach Zusatz des Inhibitors mit der vor Zusatz des Inhibitors gemessenen Aktivität vergleicht.

Ein Beispiel hierfür sind die Thiopeptolide R-Pro-X-Gly-S-Y-Z-Gly-R1 (EP 01495Q3), die von Vertebraten-Collagenase gespalten werden, sowie Acetyl-prolyl-leucyl-glycyl-[2-mercapto4-methyl-pentanoyl]-leucyl-glycyl-ethylester (US 2002/0142362), das durch bestimmte Matrixine gespalten wird, wodurch sich eine freie SH-Gruppe bildet, die mittels bekannter Verfahren, z. B. Reaktion mit DTNB, quantifiziert werden kann. Erfindungsgemäß wurde nun gefunden, dass ein Thiopeptolid der Formel Ac-Pro-Leu-Gly-[2-mercapto-4-methyl-pentanoyl]-Leu-Gly-OC2H5, worin Ac eine Acetylgruppe bedeutet, oder ein Salz davon, durch eine ADAM-TS-1 Protease oder ADAM-TS-4 Protease gespalten und so über die freie SH-Gruppe leicht nachgewiesen erden kann. Dies ist umso überraschender, als der Stand der Technik beschreibt, dass Peptide, die weniger als 16 Aminosäure-Einheiten umfassen, von ADAM-TS nicht gespalten werden können.

Ein Gegenstand der vorliegenden Erfindung betrifft daher die Verwendung eines Thiopeptolids der Formel Ac-Pro-Leu-Gly-[2-mercapto-4-methyl-pentainoyl]-Leu-Gly-OC2H5, worin Ac eine Acetylgruppe bedeutet, oder ein Salz davon, als ein Substrat für die ADAM-TS-1 Protease oder ADAM-TS-4 Protease.

Die erfindungsgemäße Verwendung eignet sich insbesondere zur Bestimmung der Aktivität einer ADAM-TS-1 oder -4 Protease zur Reinigung einer ADAM-TS-1 oder -4 Protease, zum funktionellen Klonieren einer Nukleotidsequenz kodierend für eine ADAM-TS-1 oder -4 Protease, zum Auffinden eines ADAM-TS-1 oder -4 Protease Modulators, insbesondere eines ADAM-TS-1 oder -4 Proteaseinhibitors, oder zur Beobachtung des Einsetzens oder des Verlaufs einer Krankheit, die mit einer beschädigten Gewebematrix einhergeht, insbesondere Osteoarthritis, Rheuma, Krebs, Entzündungen, Angiogenese, Zellmigration, Blutgerinnung und/oder Blutkoagulation, vor allem Osteoarthritis, Rheuma oder Krebs. Letztendlich wird bei allen diesen Methoden, z.B. während der enzymatischen Reinigung einer ADAMS-TS-1 oder -4 oder nach Klonierung eines Gens kodierend für eine ADAM-TS-1 oder -4 Protease in einen allgemein bekannten Expressionsvektor, die Aktivität der ADAM-TS-1 oder -4 Protease mit Hilfe der beschriebenen Thiopeptolide gemessen.

Die vorliegende Erfindung betrifft daher auch ein Verfahren zur Bestimmung der Aktivität einer ADAM-TS-1 oder -4 Protease, wobei das Verfahren folgende Schritte enthält:
(a) Inkubieren einer genannten ADAM-TS Protease mit einem Thiopeptolidsubstrat wie oben näher beschrieben, und
(b) Durchführen einer Aktivitätsmessung oder -bestimmung der ADAM-TS Protease.

Die Durchführung der Aktivitätsmessung oder -bestimmung erfolgt vorzugsweise spektrophotometrisch.

Bei der spektrophotometrischen Bestimmung wird in der Regel ein Nachweisreagens, hier für Thiolgruppen eingesetzt. Als vorteilhaft haben sich hierbei 4,4'-Dithiodipyridin oder insbesondere 5,5'-Dithiobis(2-nitrobenzoesäure) (DTNB), das auch als Ellmanns Reagenz bekannt ist, erwiesen. Es können jedoch auch beliebig andere, geeignete Thiol-reaktive Reagenzien eingesetzt werden, wie ein Jodacetamid, z. B. 5-Jodacetamidofluorescein (5-IAF), ein Maleimid, z. B. Fluorescein-5-maleimid, oder andere Thiol-reaktive Reagenzien wie N,N'-Didansyl-L-Cystin oder 5-(Brommethyl)fluorescein. Erhältlich sind derartige Reagenzien beispielsweise von Invitrogen GmbH, Karlsruhe.

Mit Hilfe der genannten Thiopeptolide können in einem geelgneten Testsystem besonders einfach Modulatoren der genannten ADAM-TS Proteasen aufgefunden werden. Als Modulatoren gemäß der vorliegenden Erfindung werden insbesondere Aktivatoren der ADAM-TS Protease und vor allem Inhibitoren der ADAM-TS Protease verstanden.

Ein weiterer Gegenstand der vorliegenden Erfindung bezieht sich daher auf ein Verfahren zum Auffinden eines ADAM-TS -1 oder -4 Proteasemodulators, bei dem das Verfahren folgende Schritte enthält:
(a) Inkubieren einer ADAM-TS -1 oder -4 Protease mit einem Thiopeptolidsubstrat wie oben näher beschrieben in Anwesenheit einer Testverbindung und
(b) Messen oder Bestimmen des Einflusses der Testverbindung auf die Aktivität der ADAM-TS Protease.

Die Durchführung der Aktivitätsmessung oder - bestimmung erfolgt vorzugsweise spektrophotometrisch, wie oben bereits näher beschrieben. Als Nachweisreagenz für Thiolgruppen eigenen sich wiederum die oben beschriebenen Verbindungen, besonders 4,4'-Dithiodipyridin oder 5,5'-Dithlobis(2-nitrobenzoesäure) (DTNB).

Die Testverbindung kann jede denkbare chemische, biochemisch, natürlich vorkommende oder synthetische, hoch- oder niedermolekulare Verbindung sein.

Besonders vorteilhaft ist es, wenn die Testverbindungen in Form einer chemischen Verbindungsbibilothek zur Verfügung gestellt wird, aus der dann mit Hilfe des erfindungsgemäßen Verfahrens die gewünschte Verbindung, z. B. ein Inhibitor der getesteten ADAM-TS Protease, aufgefunden werden kann.

In einer weiteren bevorzugten Ausführungsform wird das erfindungsgemäße Verfahren auf einem Array durchgeführt, welcher das Auffinden und Isolieren der gewünschten Verbindung besonders erleichtert. Der Einsatz eines Roboters für die Durchführung des erfindungsgemäßen Verfahrens führt ebenso zu einer weiteren Erleichterung und zu einer Erhöhung des Durchsatzes und ist daher besonders vorteilhaft. Mit Hilfe der Mikrofluidtechnik ggf. in Verbindung mit miniaturisierten Plattenvertiefungen ("wells") gelingt auch eine Verkleinerung und weitere Automatisierung des Testsystems, was wiederum besonders vorteilhaft ist. Im allgemeinen wird das Verfahren in einem Hochdurchsatzscreening nach einem ADAM-TS Proteasemodulator, insbesondere einem ADAM-TS Proteaseinhibitors eingesetzt.

Die vorliegende Beschreibung offenbart auch die Herstellung eines Medikamentes, das folgende Schritte enthält:
(a) Durchführen des oben genannten Verfahrens zum Auffinden eines ADAM-TS-1 oder -4 Modulators, insbesondere eines Inhibitors,
(b) Isolieren einer gemäß Schritt (a) aufgefundenen geeigneten Testsubstanz und
(c) Formulieren der gemäß Schritt (b) Isollerten Testsubstanz mit einem oder mehreren pharmazeutisch akzeptablen Trägem oder Hilfssubstanzen.

Die mit Hilfe des erfindungsgemäßen Verfahrens aufgefundenen pharmazeutisch wirksamen Verbindungen, vorzugsweise Inhibitoren, eignen sich vor allem zur Behandlung einer Krankheit, die mit einer beschädigten Gewebematrix einhergeht, insbesondere Osteoarthritis, Rheuma, Krebs, Entzündungen, Angiogenese, Zellmigration, Blutgerinnung und/oder Blutkoagulation, vor allem Osteoarthritis, Rheuma oder Krebs.

Als pharmazeutisch akzeptabler Träger oder Hilfssubstanz eignen sich alle bekannten Mittel, die zur Formulierung von Arzneimitteln üblicherweise eingesetzt werden.

Beispiele sind eine Natriumchloridlösung, insbesondere eine isotonische Salzlösung (0,9%ige Natriumchloridlösung), demineralisiertes Wasser, Stabilisatoren wie Protease- oder Nukleaseinhibitoren, vorzugsweise Aprotinin, ε-Aminokapronsäure oder Pepstatin A, oder Maskierungsagentien wie EDTA, Gelformulierungen wie weißes Vaselin, niederviskoses Paraffin und/oder gelbes Wachs, abhängig von der Art der Verabreichung.

Weitere geeignete Additive sind beispielsweise Detergentien wie Triton X-100 oder Natriumdeoxycholat aber auch Polyole wie Polyethylenglycol oder Glycerin, Zucker -wie Sukrose oder Glukose, zwitterionische Verbindungen wie Aminosäuren, z. B. Glycin oder insbesondere Taurin oder Betain und/oder ein Protein wie Rinderserumalbumin oder humanes Serumalbumin. Detergentien, Polyole und/oder zwitterionische Verbindungen sind besonders bevorzugt.

Die physiologische Pufferlösung hat vorzugsweise einen pH von ungefähr 6.0-8.0, insbesondere einen pH von ungefähr 6.8-7.8, vor allem einen pH von ungefähr 7.4 und/oder eine Osmolarität von ungefähr 200-400 Mililosmol/Liter, vorzugsweise ungefähr 290-310 Milliosmol/Liter. Der pH-Wert des Medikamentes wird im allgemeinen mit Hilfe geeigneter organischer oder anorganischer Puffer eingestellt, z. B. vorzugsweise mit Hilfe von Phosphatpuffer, Tris-Puffer (Tris(hydroxymethyl)aminomethan), HEPES-Puffer ([4-(2-Hydroxyethyl)piperazino]ethansulphonsäure) oder MOPS-Puffer (3-Morpholino-1-propansulphonsäure). Die Wahl des entsprechenden Puffers hängt im allgemeinen von der gewünschten Puffermolarität ab. Phosphatpuffer ist z. B. für Injektions- und Infusionslösungen geeignet.

Ein weiterer Gegenstand der vorliegenden Erfindung ist ein Kit, der auf dem erfindungsgemäßen Thiopeptosubstrate wie oben näher beschrieben und einer ADAM-TS -1 oder -4 Protease wie oben beschrieben beruht und gegebenenfalls einen oder mehrere Puffer, z. B. TNCB-Puffer (siehe Beispiel), enthält.

Der Puffer dient als Stabilisierungs- oder Reaktionsmedium zur Durchführung des erfindungsgemäßen Verfahrens. Vorzugsweise enthält der Kit zusätzlich ein Nachweisreagenz für Thiolgruppen, z. B. mindestens eines der oben beschriebenen Nachweisreagenzien, insbesondere 4,4'-Dithiodipyridin und/oder 5,5'-Dithiobis(2-nitrobenzoesäure) (DTNB). Eine weitere Komponente des Kits kann eine Gebrauchsanleitung zur Durchführung des erfindungsgemäßen Verfahrens, insbesondere des Aktivitätstest, sein.

Die folgenden Ausführungen und Beispiele sollen die Erfindung näher erläutern ohne sie darauf zu beschränken:

### Sequenzprotokoll

SEQ ID NO: 1 entspricht dem Thiopeptolid gemäß Formel (I)
SEQ ID NO: 2 stellt die Aminosäuresequenz des Aggrecan Kemproteinvorläufers (Knorpel-specifisches Proteoglycan Kernprotein, CSPCP oder Chondroitinsulfate Proteoglycan Kernprotein 1) dar.

### Beispiele

### BEISPIEL 1 (VERGLEICHSBEISPIEL)

### Testbedingungen

Die katalytischen Domänen des humanen rekombinanten MMP-3 und MMP-8 Proteins, MMP-3cd und MMP-8cd, sind käuflich bei z. B. Biomol International L. P. Pennsylvania, USA; Katalognummer SE-109 bzw. SE-255 erhältlich. Die verkürzten Formen der ADAM-TS1 und ADAM-TS4 Protease sind ebenso käuflich bei z. B. Invitek, Gesellschaft für Biotechnik & Biodesign mbH, Berlin, Deutschland, Katalognummer 30400402 bzw. 30400102 erhältlich.

### Herstellung der Puffer bzw. Lösungen

TNCB Puffer:
   100mM Tris-(hydroxymethyl)-aminomethan, eingestellt mit HCl auf pH 7.5
   100mM NaCl
   10mM CaCl₂·2H₂O
   0.015% Brij 35
Substrat-Lösung:
   10mM Substrate (siehe Tabelle) in DMSO. Unmittelbar vor Gebrauch wurden 2µL der Substratstammlösung mit 130µL H₂O verdünnt.
Enzym-Lösung:
   MMP-3cd (2.3µg/mL), MMP-8cd (0.6µg/mL), ADAMTS-1 (2.3µg/mL) und ADAMTS-4 (3.3µg/mL) wurden mit TNCB-Puffer verdünnt.

### Durchführung des Tests

Es wurden 10µL Enzymlösung und 10µL H₂O vorgelegt und die Reaktion durch Zugabe von 10 µL Substratlösung gestartet.

### Fluorometrische Analyse

Die Messung der Fluoreszenz erfolgte in dem Fluoreszenzgerät TECAN Spectrafluor Plus; Exitation/Emmission (siehe Tabelle). Hierbei wurde die Fluoreszenz für jeweils 5 Minuten gemessen.

### Ergebnisse zum Vergleichsbeispiel 1

| | + : Anstieg im optischen Signal wurde über 5 Minuten beobachtet | | | | | |
|---|---|---|---|---|---|---|
| Fluorimetrische Substanz | | λₑₓ/λₑₘ | ADAMTS-4 | ADAMTS-1 | MMP-3cd | MMP-8cd |
| Dnp-P-L-G-L-W-A-R-NH2 | Bachem: M-1855 | 280/355 | - | - | + | - |
| Mca-G-K-P-I-L F-F-R-L-K-(Dnp)-R-NH2 | Sigma: M-0938 | 330/390 | - | - | + | - |
| Mca-P-L-A-Q-A-V-Dap(Dnp)-R-S-S-S-R-NH2 | Bachem: M-2255/ R&D: ES003 | 330/390 | - | - | + | - |
| Mcä-P-L-G-L-Dap(Dnp)-A-R-NH2 | Bachem: M-1895 | 330/390 | - | - | + | + |
| Mca-P-β-cyclohexyl-A-G-Nva-H-A-Dpa-NH2 | Calbiochem: 444235 | 330/390 | - | - | + | + |
| Mca-R-P-K-P-V-E-Nval-W-R-K-(Dnp)-NH2 | Bachem: M-2110/ R&D: ES002 | 330/390 | - | - | + | - |
| Mca-R-P-L-A-L-W-R-Dap(Dnp)-NH2 | Bachem: M-2390 | 330/390 | - | - | + | + |
| Mca-P-L-A-C(Mob)-W-A-R-Dap(Dnp)-NH2 | Bachem: M-2510 | 330/390 | - | - | + | + |
| Mca-R-P-K-P-Y-A-Nva-Met-K(Dnp)-NH2 | Bachem: M-2105 | 330/390 | - | - | + | + |
| Mca-P-L-A-Nva-Dap(Dnp)-A-R-NH2 | Bachem: M-2520 | 330/390 | - | - | + | + |
| NBD-eAhx-R-P-K-P-L-A-Nva-W-K(DMACA)-NH2 | Bachem: M-2300 | 350/465 | - | - | + | + |
| Dnp-P-β-cyclohexyl-A-G-C(Me)-H-A-K-(N-Me-Abz)-NH2 | Bachem: M-2055 | 365/450 | - | - | + | + |

Erwartungsgemäß und gemäß dem Stand der Technik wurden die MMP Substrate nicht von ADAMTS umgesetzt.

### BEISPIEL 2

### Herstellung der Puffer bzw. Lösungen

### TNCB Puffer (siehe Beispiel 1)

### Enzym-Lösung:

ADAMTS (Invitek Gesellschaft für Biotechnik & Biodesign mbH, Berlin, Deutschland) Es wurden 5µg ADAMTS-1 mit 2200µL, bzw., 5µg ADAMTS-4 mit 600µl TNCB Puffer verdünnt.

### Substrat- Lösung

1) DTNB Lösung (5,5'-Dithio-bis(2-Nitrobenzoesäure):
   Es wurde eine 40mM Stammlösung in DMSO hergestellt:
      Anschließend wurden 27.5µL DTNB Stammlösung mit 522µL Wasser verdünnt.
2) Thiopeptilid- Lösung: (Bachem Distribution Services GmbH, Weil am Rhein, Deutschland):
   Es wurde eine 100mM Stammlösung in DMSO hergestellt. Zum Gebrauch wurden 55µL Thiopeptilid Stammlösung mit 500µL TNCB Puffer verdünnt.
   Unmittelbar vor dem Gebrauch wurden 550µl DTNB mit 550µl Thiopeptilid gemischt.

### Durchführung des Tests

Die Messungen wurden in 96-Multiwellplatten (half area plates, flat bottom, clear, polystyrene, No.3695) durchgeführt (Coming Costar, Acton, USA).

Es wurden 10µL Enzymlösung und 10µL H₂O vorgelegt und die Reaktion durch Zugabe von 10 µL Substratlösung gestartet

### Colorimetrische Analyse

Mikrotlterplattenphotometer: Molecular Devices Sunnyvale, USA. SpectraMax 190.
Die Absorption wurde für 5min bei einer Wellenlänge von 415nm beobachtet.

### Ergebnisse zu Beispiel 2

| Colorimetrische Substrate in der Ellmans Reaktion | | λ (nm) | ADAMTS-4 | ADAMTS-1 | MMP-3cd | MMP-8cd |
|---|---|---|---|---|---|---|
| Ac-P-L-G-[(S)-2-mercapto-4-methylpentanoyl]-L-G-OEt | Bachem: H-7145 | 415 | + | + | + | + |
| Ac-P-L-A-[(S)-2-mercapto-pentanoyl]-W-NH2 | Bachem: H-1326 | 415 | - | - | + | + |

| Verbindung 1 Bachem: H-7145 | Verbindung 2 (Vergleichsbeispiel) Bachem: H-1326 |
|---|---|
| (Synonyme) | (Synonyme) |
| Ac-P-L-G-^{S}L-L-G-OEt | Ac-P-L-A-^{S}Nva-W-NH2 |
| Ac-P-L-G-[(S)-2-mercapto-4-methylpentanoyl]-L-G-OEt | Ao-P-L-A-[(S)-2-mercapto-pentanoyl]-W-NH2 |
| Ao-P-L-G-Sch[CH2CH(CH3)2]-CO-L-G-OC2H5 | Ac-P-L-A-[2-mercapto-pentanoyl]-W-NH2 |
| Ac-P-L-G-[2-mereapto-4-methyl-pentanoyl]-L-G-OC2H5 | |

### Strukturformel der Verbindung 1

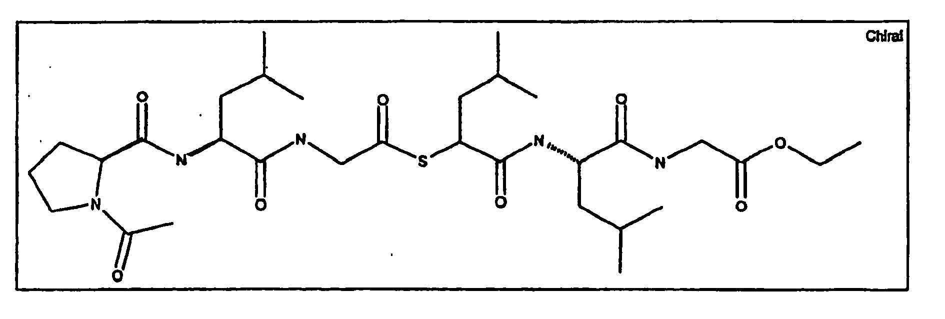

### Strukturformel der Verbindung 2 (Vergleichsbeispiel)

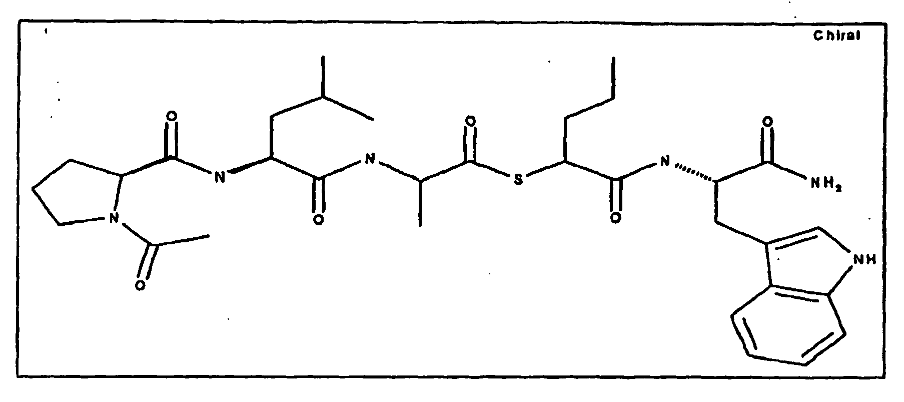

Verbindung 2 wurde von ADAMTS nicht umgesetzt. Überraschenderweise wurde jedoch Verbindung 1 von ADAMTS umgesetzt.
**SEQ ID NO: 1**
**Xaa-Pro-Xaa-Gly-Xaa-Xaa-Gly-Xaa**

## Patentansprüche

1. Verwendung eines Thiopeptolids der Formel
Ac-Pro-Leu-Gly-[2-mercapto-4-methyl-pentanoyl]-Leu-Gly-OC₂H₅,
worin Ac eine Acetylgruppe bedeutet
oder ein Salz davon,
als ein Substrat für die ADAM-TS-1 Protease oder ADAM-TS-4 Protease.

2. Verfahren zur Bestimmung der Aktivität einer ADAM-TS-1 Protease oder ADAM-TS-4 Protease, **dadurch gekennzeichnet, dass** das Verfahren folgende Schritte enthält:
(a) Inkubieren einer genannten ADAM-TS Protease mit einem Thiopeptolidsubstrat gemäß Anspruch 1, und
(b) Durchführen einer Aktivitätsmessung oder- bestimmung der ADAM-TS Protease.

3. Verfahren nach Anspruch 2, **dadurch gekennzeichnet, dass** die Aktivität der ADAM-TS Protease spektrophotometrisch gemessen oder bestimmt wird.

4. Verfahren zum Auffinden eines ADAM-TS-1 Proteasemodulators oder eines ADAM-TS-4 Proteasemodulators, insbesondere eines entsprechenden Inhibitors, **dadurch gekennzeichnet, dass** das Verfahren folgende Schritte enthält:
(a) Inkubieren einer ADAM-TS-1 Protease oder ADAM-TS-4 Protease mit einem Thiopeptolidsubstrat gemäß Anspruch 1 in Anwesenheit einer Testverbindung und
(b) Messen oder Bestimmen des Einflusses der Testverbindung auf die Aktivität der ADAM-TS Protease.

5. Verfahren nach Anspruch 4, **dadurch gekennzeichnet, dass** die Aktivität der ADAM-TS Protease spektrophotometrisch gemessen oder bestimmt wird.

6. Verfahren nach Anspruch 4 oder 5, **dadurch gekennzeichnet, dass** die Testverbindung in Form einer chemischen Verbindungsbibliothek zur Verfügung gestellt wird.

7. Verfahren nach mindestens einem der Ansprüche 4-6, **dadurch gekennzeichnet, dass** das Verfahren auf einem Array durchgeführt wird.

8. Verfahren nach mindestens einem der Ansprüche 4-7, **dadurch gekennzeichnet, dass** das Verfahren mittels eines Roboters durchgeführt wird.

9. Verfahren nach mindestens einem der Ansprüche 4-8, **dadurch gekennzeichnet, dass** das Verfahren mit Hilfe der Mikrofluidtechnik durchgeführt wird.

10. Kit enthaltend ein Thiopeptolidsubstrat gemäß Anspruch 1, eine ADAM-TS-1 Protease und/oder ADAM-TS-4 Protease und gegebenenfalls einen oder mehrere Puffer.

## Claims

1. The use of a thiopeptolide of the formula
Ac-Pro-Leu-Gly-[2-mercapto-4-methyl-pentanoyl]-Leu-Gly-OC₂H₅,
in which Ac is an acetyl group
or a salt thereof,
as a substrate for the ADAM-TS-1 protease or ADAM-TS-4 protease.

2. A method for determining the activity of an ADAM-TS-1 protease or ADAM-TS-4 protease, **characterized in that** the method comprises the following steps:
(a) incubating a recited ADAM-TS protease with a thiopeptolide substrate according to claim 1, and
(b) carrying out an activity measurement or determination of the ADAM-TS protease.

3. The method as claimed in claim 2, **characterized in that** the activity of the ADAM-TS protease is measured or determined by spectrophotometry.

4. A method for finding an ADAM-TS-1 protease modulator or an ADAM-TS-4 protease modulator, in particular a corresponding inhibitor, **characterized in that** the method comprises the following steps:
(a) incubating an ADAM-TS-1 protease or ADAM-TS-4 protease with a thiopeptolide substrate according to claim 1 in the presence of a test compound and
(b) measuring or determining the influence of the test compound on the activity of the ADAM-TS protease.

5. The method as claimed in claim 4, **characterized in that** the activity of the ADAM-TS protease is measured or determined by spectrophotometry.

6. The method as claimed in claim 4 or 5, **characterized in that** the test compound is made available in the form of a chemical compound library.

7. The method as claimed in at least one of claims 4-6, **characterized in that** the method is carried out on an array.

8. The method as claimed in at least one of claims 4-7, **characterized in that** the method is carried out by means of a robot.

9. The method as claimed in at least one of claims 4-8, **characterized in that** the method is carried out with the aid of microfluidic technology.

10. A kit comprising a thiopeptolide substrate according to claim 1, an ADAM-TS-1 protease and/or ADAM-TS-4 protease and where appropriate one or more buffers.

## Revendications

1. Utilisation d'un thiopeptolide de formule Ac-Pro-Leu-Gly-[2-mercapto-4-méthylpentanoyl]-Leu-Gly-OC₂H₅, où Ac signifie un groupe acétyle ou un sel de celui-ci, comme substrat pour la protéase ADAM-TS-1 ou la protéase ADAM-TS-4.

2. Procédé pour la détermination de l'activité d'une protéase ADAM-TS-1 ou d'une protéase ADAM-TS-4, **caractérisé en ce que** le procédé comporte les étapes suivantes :
(a) incubation d'une protéase ADAM-TS mentionnée avec un substrat thiopeptolide selon la revendication 1, et
(b) réalisation d'une mesure ou d'une détermination de l'activité de la protéase.

3. Procédé selon la revendication 2, **caractérisé en ce que** l'activité de la protéase ADAM-TS est mesurée ou déterminée par spectrophotométrie.

4. Procédé pour trouver un modulateur de la protéase ADAM-TS-1 ou un modulateur de la protéase ADAM-TS-4, en particulier un inhibiteur correspondant, **caractérisé en ce que** le procédé comporte les étapes suivantes :
(a) incubation d'une protéase ADAM-TS-1 ou d'une protéase ADAM-TS-4 avec un substrat thiopeptolide selon la revendication 1 en présence d'un composé test et
(b) mesure ou détermination de l'influence du composé test sur l'activité de la protéase ADAM-TS.

5. Procédé selon la revendication 4, **caractérisé en ce que** l'activité de la protéase ADAM-TS est mesurée ou déterminée par spectrophotométrie.

6. Procédé selon la revendication 4 ou 5, **caractérisé en ce que** le composé test est mis à disposition sous forme d'une bibliothèque de composés chimiques.

7. Procédé selon au moins l'une quelconque des revendications 4 à 6, **caractérisé en ce que** le procédé est réalisé sur une matrice.

8. Procédé selon au moins l'une quelconque des revendications 4 à 7, **caractérisé en ce que** le procédé est réalisé au moyen d'un robot.

9. Procédé selon au moins l'une quelconque des revendications 4 à 8, **caractérisé en ce que** le procédé est réalisé à l'aide de la technique microfluidique.

10. Kit contenant un substrat thiopeptolide selon la revendication 1, une protéase ADAM-TS-1 et/ou une protéase ADAM-TS-4 et le cas échéant un ou plusieurs tampons.


### SEQUENZPROTOKOLL

<110> Sanofi-Aventis Deutschland GmbH
<120> Verwendung von Thiopeptoliden zur Aktivitätsbestimmung von ADAM-TS Proteasen
<130> DE 2005/006
<140>
   <141>
<160> 2
<170> PatentIn Ver. 2.1
<210> 1
   <211> 8
   <212> PRT
   <213> Künstliche Sequenz
<220>
   <223> Beschreibung der künstlichen Sequenz: Peptid
<400> 1
   Xaa Pro Xaa Gly Xaa Xaa Gly Xaa
    1               5
<210> 2
   <211> 2415
   <212> PRT
   <213> Homo sapiens
<400> 2
   Met Thr Thr Leu Leu Trp Val Phe Val Thr Leu Arg Val Ile Thr Ala
    1               5                  10                  15
   Ala Val Thr Val Glu Thr Ser Asp His Asp Asn Ser Leu Ser Val Ser
               20                  25                  30
   Ile Pro Gln Pro Ser Pro Leu Arg Val Leu Leu Gly Thr Ser Leu Thr
           35                  40                  45
   Ile Pro Cys Tyr Phe Ile Asp Pro Met His Pro Val Thr Thr Ala Pro
       50                  55                  60
   Ser Thr Ala Pro Leu Ala Pro Arg Ile Lys Trp Ser Arg Val Ser Lys
   65 70 75 80
   Glu Lys Glu Val Val Leu Leu Val Ala Thr Glu Gly Arg Val Arg Val
   85 90 95
   Asn Ser Ala Tyr Gln Asp Lys Val Ser Leu Pro Asn Tyr Pro Ala Ile
   100 105 110
   Pro Ser Asp Ala Thr Leu Glu Val Gln Ser Leu Arg Ser Asn Asp Ser
   115 120 125
   Gly Val Tyr Arg Cys Glu Val Met His Gly Ile Glu Asp Ser Glu Ala
   130 135 140
   Thr Leu Glu Val Val Val Lys Gly Ile Val Phe His Tyr Arg Ala Ile
   145 150 155 160
   Ser Thr Arg Tyr Thr Leu Asp Phe Asp Arg Ala Gln Arg Ala Cys Leu
   165 170 175
   Gln Asn Ser Ala Ile Ile Ala Thr Pro Glu Gln Leu Gln Ala Ala Tyr
   180 185 190
   Glu Asp Gly Phe His Gln Cys Asp Ala Gly Trp Leu Ala Asp Gln Thr
   195 200 205
   Val Arg Tyr Pro Ile His Thr Pro Arg Glu Gly Cys Tyr Gly Asp Lys
   210 215 220
   Asp Glu Phe Pro Gly Val Arg Thr Tyr Gly Ile Arg Asp Thr Asn Glu
   225 230 235 240
   Thr Tyr Asp Val Tyr Cys Phe Ala Glu Glu Met Glu Gly Glu Val Phe
   245 250 255
   Tyr Ala Thr Ser Pro Glu Lys Phe Thr Phe Gln Glu Ala Ala Asn Glu
   260 265 270
   Cys Arg Arg Leu Gly Ala Arg Leu Ala Thr Thr Gly His Val Tyr Leu
   275 280 285
   Ala Trp Gln Ala Gly Met Asp Met Cys Ser Ala Gly Trp Leu Ala Asp
   290 295 300
   Arg Ser Val Arg Tyr Pro Ile Ser Lys Ala Arg Pro Asn Cys Gly Gly
   305 310 315 320
   Asn Leu Leu Gly Val Arg Thr Val Tyr Val His Ala Asn Gln Thr Gly
   325 330 335
   Tyr Pro Asp Pro Ser Ser Arg Tyr Asp Ala Ile Cys Tyr Thr Gly Glu
   340 345 350
   Asp Phe Val Asp Ile Pro Glu Asn Phe Phe Gly Val Gly Gly Glu Glu
   355 360 365
   Asp Ile Thr Val Gln Thr Val Thr Trp Pro Asp Met Glu Leu Pro Leu
   370 375 380
   Pro Arg Asn Ile Thr Glu Gly Glu Ala Arg Gly Ser Val Ile Leu Thr
   385 390 395 400
   Val Lys Pro Ile Phe Glu Val Ser Pro Ser Pro Leu Glu Pro Glu Glu
   405 410 415
   Pro Phe Thr Phe Ala Pro Glu Ile Gly Ala Thr Ala Phe Ala Glu Val
   420 425 430
   Glu Asn Glu Thr Gly Glu Ala Thr Arg Pro Trp Gly Phe Pro Thr Pro
   435 440 445
   Gly Leu Gly Pro Ala Thr Ala Phe Thr Ser Glu Asp Leu Val Val Gln
   450 455 460
   Val Thr Ala Val Pro Gly Gln Pro His Leu Pro Gly Gly Val Val Phe
   465 470 475 480
   His Tyr Arg Pro Gly Pro Thr Arg Tyr Ser Leu Thr Phe Glu Glu Ala
   485 490 495
   Gln Gln Ala Cys Pro Gly Thr Gly Ala Val Ile Ala Ser Pro Glu Gln
   500 505 510
   Leu Gln Ala Ala Tyr Glu Ala Gly Tyr Glu Gln Cys Asp Ala Gly Trp
   515 520 525
   Leu Arg Asp Gln Thr Val Arg Tyr Pro Ile Val Ser Pro Arg Thr Pro
   530 535 540
   Cys Val Gly Asp Lys Asp Ser Ser Pro Gly Val Arg Thr Tyr Gly Val
   545 550 555 560
   Arg Pro Ser Thr Glu Thr Tyr Asp Val Tyr Cys Phe Val Asp Arg Leu
   565 570 575
   Glu Gly Glu Val Phe Phe Ala Thr Arg Leu Glu Gln Phe Thr Phe Gln
   580 585 590
   Glu Ala Leu Glu Phe Cys Glu Ser His Asn Ala Thr Ala Thr Thr Gly
   595 600 605
   Gln Leu Tyr Ala Ala Trp Ser Arg Gly Leu Asp Lys Cys Tyr Ala Gly
   610 615 620
   Trp Leu Ala Asp Gly Ser Leu Arg Tyr Pro Ile Val Thr Pro Arg Pro
   625 630 635 640
   Ala Cys Gly Gly Asp Lys Pro Gly Val Arg Thr Val Tyr Leu Tyr Pro
   645 650 655
   Asn Gln Thr Gly Leu Pro Asp Pro Leu Ser Arg His His Ala Phe Cys
   660 665 670
   Phe Arg Gly Ile Ser Ala Val Pro Ser Pro Gly Glu Glu Glu Gly Gly
   675 680 685
   Thr Pro Thr Ser Pro Ser Gly Val Glu Glu Trp Ile Val Thr Gln Val
   690 695 700
   Val Pro Gly Val Ala Ala Val Pro Val Glu Glu Glu Thr Thr Ala Val
   705 710 715 720
   Pro Ser Gly Glu Thr Thr Ala Ile Leu Glu Phe Thr Thr Glu Pro Glu
   725 730 735
   Asn Gln Thr Glu Trp Glu Pro Ala Tyr Thr Pro Val Gly Thr Ser Pro
   740 745 750
   Leu Pro Gly Ile Leu Pro Thr Trp Pro Pro Thr Gly Ala Glu Thr Glu
   755 760 765
   Glu Ser Thr Glu Gly Pro Ser Ala Thr Glu Val Pro Ser Ala Ser Glu
   770 775 780
   Glu Pro Ser Pro Ser Glu Val Pro Phe Pro Ser Glu Glu Pro Ser Pro
   785 790 795 800
   Ser Glu Glu Pro Phe Pro Ser Val Arg Pro Phe Pro Ser Val Glu Leu
   805 810 815
   Phe Pro Ser Glu Glu Pro Phe Pro Ser Lys Glu Pro Ser Pro Ser Glu
   820 825 830
   Glu Pro Ser Ala Ser Glu Glu Pro Tyr Thr Pro Ser Pro Pro Glu Pro
   835 840 845
   Ser Trp Thr Glu Leu Pro Ser Ser Gly Glu Glu Ser Gly Ala Pro Asp
   850 855 860
   Val Ser Gly Asp Phe Thr Gly Ser Gly Asp Val Ser Gly His Leu Asp
   865 870 875 880
   Phe Ser Gly Gln Leu Ser Gly Asp Arg Ala Ser Gly Leu Pro Ser Gly
   885 890 895
   Asp Leu Asp Ser Ser Gly Leu Thr Ser Thr Val Gly Ser Gly Leu Thr
   900 905 910
   Val Glu Ser Gly Leu Pro Ser Gly Asp Glu Glu Arg Ile Glu Trp Pro
   915 920 925
   Ser Thr Pro Thr Val Gly Glu Leu Pro Ser Gly Ala Glu Ile Leu Glu
   930 935 940
   Gly Ser Ala Ser Gly Val Gly Asp Leu Ser Gly Leu Pro Ser Gly Glu
   945 950 955 960
   Val Leu Glu Thr Ser Ala Ser Gly Val Gly Asp Leu Ser Gly Leu Pro
   965 970 975
   Ser Gly Glu Val Leu Glu Thr Thr Ala Pro Gly Val Glu Asp Ile Ser
   980 985 990
   Gly Leu Pro Ser Gly Glu Val Leu Glu Thr Thr Ala Pro Gly Val Glu
   995 1000 1005
   Asp Ile Ser Gly Leu Pro Ser Gly Glu Val Leu Glu Thr Thr Ala Pro
   1010 1015 1020
   Gly Val Glu Asp Ile Ser Gly Leu Pro Ser Gly Glu Val Leu Glu Thr
   1025 1030 1035 1040
   Thr Ala Pro Gly Val Glu Asp Ile Ser Gly Leu Pro Ser Gly Glu Val
   1045 1050 1055
   Leu Glu Thr Thr Ala Pro Gly Val Glu Asp Ile Ser Gly Leu Pro Ser
   1060 1065 1070
   Gly Glu Val Leu Glu Thr Ala Ala Pro Gly Val Glu Asp Ile Ser Gly
   1075 1080 1085
   Leu Pro Ser Gly Glu Val Leu Glu Thr Ala Ala Pro Gly Val Glu Asp
   1090 1095 1100
   Ile Ser Gly Leu Pro Ser Gly Glu Val Leu Glu Thr Ala Ala Pro Gly
   1105 1110 1115 1120
   Val Glu Asp Ile Ser Gly Leu Pro Ser Gly Glu Val Leu Glu Thr Ala
   1125 1130 1135
   Ala Pro Gly Val Glu Asp Ile Ser Gly Leu Pro Ser Gly Glu Val Leu
   1140 1145 1150
   Glu Thr Ala Ala Pro Gly Val Glu Asp Ile Ser Gly Leu Pro Ser Gly
   1155 1160 1165
   Glu Val Leu Glu Thr Ala Ala Pro Gly Val Glu Asp Ile Ser Gly Leu
   1170 1175 1180
   Pro Ser Gly Glu Val Leu Glu Thr Ala Ala Pro Gly Val Glu Asp Ile
   1185 1190 1195 1200
   Ser Gly Leu Pro Ser Gly Glu Val Leu Glu Thr Ala Ala Pro Gly Val
   1205 1210 1215
   Glu Asp Ile Ser Gly Leu Pro Ser Gly Glu Val Leu Glu Thr Ala Ala
   1220 1225 1230
   Pro Gly Val Glu Asp Ile Ser Gly Leu Pro Ser Gly Glu Val Leu Glu
   1235 1240 1245
   Thr Ala Ala Pro Gly Val Glu Asp Ile Ser Gly Leu Pro Ser Gly Glu
   1250 1255 1260
   Val Leu Glu Thr Ala Ala Pro Gly Val Glu Asp Ile Ser Gly Leu Pro
   1265 1270 1275 1280
   Ser Gly Glu Val Leu Glu Thr Thr Ala Pro Gly Val Glu Glu Ile Ser
   1285 1290 1295
   Gly Leu Pro Ser Gly Glu Val Leu Glu Thr Thr Ala Pro Gly Val Asp
   1300 1305 1310
   Glu Ile Ser Gly Leu Pro Ser Gly Glu Val Leu Glu Thr Thr Ala Pro
   1315 1320 1325
   Gly Val Glu Glu Ile Ser Gly Leu Pro Ser Gly Glu Val Leu Glu Thr
   1330 1335 1340
   Ser Thr Ser Ala Val Gly Asp Leu Ser Gly Leu Pro Ser Gly Gly Glu
   1345 1350 1355 1360
   Val Leu Glu Ile Ser Val Ser Gly Val Glu Asp Ile Ser Gly Leu Pro
   1365 1370 1375
   Ser Gly Glu Val Val Glu Thr Ser Ala Ser Gly Ile Glu Asp Val Ser
   1380 1385 1390
   Glu Leu Pro Ser Gly Glu Gly Leu Glu Thr Ser Ala Ser Gly Val Glu
   1395 1400 1405
   Asp Leu Ser Arg Leu Pro Ser Gly Glu Glu Val Leu Glu Ile Ser Ala
   1410 1415 1420
   Ser Gly Phe Gly Asp Leu Ser Gly Val Pro Ser Gly Gly Glu Gly Leu
   1425 1430 1435 1440
   Glu Thr Ser Ala Ser Glu Val Gly Thr Asp Leu Ser Gly Leu Pro Ser
   1445 1450 1455
   Gly Arg Glu Gly Leu Glu Thr Ser Ala Ser Gly Ala Glu Asp Leu Ser
   1460 1465 1470
   Gly Leu Pro Ser Gly Lys Glu Asp Leu Val Gly Ser Ala Ser Gly Asp
   1475 1480 1485
   Leu Asp Leu Gly Lys Leu Pro Ser Gly Thr Leu Gly Ser Gly Gln Ala
   1490 1495 1500
   Pro Glu Thr Ser Gly Leu Pro Ser Gly Phe Ser Gly Glu Tyr Ser Gly
   1505 1510 1515 1520
   Val Asp Leu Gly Ser Gly Pro Pro Ser Gly Leu Pro Asp Phe Ser Gly
   1525 1530 1535
   Leu Pro Ser Gly Phe Pro Thr Val Ser Leu Val Asp Ser Thr Leu Val
   1540 1545 1550
   Glu Val Val Thr Ala Ser Thr Ala Ser Glu Leu Glu Gly Arg Gly Thr
   1555 1560 1565
   Ile Gly Ile Ser Gly Ala Gly Glu Ile Ser Gly Leu Pro Ser Ser Glu
   1570 1575 1580
   Leu Asp Ile Ser Gly Arg Ala Ser Gly Leu Pro Ser Gly Thr Glu Leu
   1585 1590 1595 1600
   Ser Gly Gln Ala Ser Gly Ser Pro Asp Val Ser Gly Glu Ile Pro Gly
   1605 1610 1615
   Leu Phe Gly Val Ser Gly Gln Pro Ser Gly Phe Pro Asp Thr Ser Gly
   1620 1625 1630
   Glu Thr Ser Gly Val Thr Glu Leu Ser Gly Leu Ser Ser Gly Gln Pro
   1635 1640 1645
   Gly Val Ser Gly Glu Ala Ser Gly Val Leu Tyr Gly Thr Ser Gln Pro
   1650 1655 1660
   Phe Gly Ile Thr Asp Leu Ser Gly Glu Thr Ser Gly Val Pro Asp Leu
   1665 1670 1675 1680
   Ser Gly Gln Pro Ser Gly Leu Pro Gly Phe Ser Gly Ala Thr Ser Gly
   1685 1690 1695
   Val Pro Asp Leu Val Ser Gly Thr Thr Ser Gly Ser Gly Glu Ser Ser
   1700 1705 1710
   Gly Ile Thr Phe Val Asp Thr Ser Leu Val Glu Val Ala Pro Thr Thr
   1715 1720 1725
   Phe Lys Glu Glu Glu Gly Leu Gly Ser Val Glu Leu Ser Gly Leu Pro
   1730 1735 1740
   Ser Gly Glu Ala Asp Leu Ser Gly Lys Ser Gly Met Val Asp Val Ser
   1745 1750 1755 1760
   Gly Gln Phe Ser Gly Thr Val Asp Ser Ser Gly Phe Thr Ser Gln Thr
   1765 1770 1775
   Pro Glu Phe Ser Gly Leu Pro Ser Gly Ile Ala Glu Val Ser Gly Glu
   1780 1785 1790
   Ser Ser Arg Ala Glu Ile Gly Ser Ser Leu Pro Ser Gly Ala Tyr Tyr
   1795 1800 1805
   Gly Ser Gly Thr Pro Ser Ser Phe Pro Thr Val Ser Leu Val Asp Arg
   1810 1815 1820
   Thr Leu Val Glu Ser Val Thr Gln Ala Pro Thr Ala Gln Glu Ala Gly
   1825 1830 1835 1840
   Glu Gly Pro Ser Gly Ile Leu Glu Leu Ser Gly Ala His Ser Gly Ala
   1845 1850 1855
   Pro Asp Met Ser Gly Glu His Ser Gly Phe Leu Asp Leu Ser Gly Leu
   1860 1865 1870
   Gln Ser Gly Leu Ile Glu Pro Ser Gly Glu Pro Pro Gly Thr Pro Tyr
   1875 1880 1885
   Phe Ser Gly Asp Phe Ala Ser Thr Thr Asn Val Ser Gly Glu Ser Ser
   1890 1895 1900
   Val Ala Met Gly Thr Ser Gly Glu Ala Ser Gly Leu Pro Glu Val Thr
   1905 1910 1915 1920
   Leu Ile Thr Ser Glu Phe Val Glu Gly Val Thr Glu Pro Thr Ile Ser
   1925 1930 1935
   Gln Glu Leu Gly Gln Arg Pro Pro Val Thr His Thr Pro Gln Leu Phe
   1940 1945 1950
   Glu Ser Ser Gly Lys Val Ser Thr Ala Gly Asp Ile Ser Gly Ala Thr
   1955 1960 1965
   Pro Val Leu Pro Gly Ser Gly Val Glu Val Ser Ser Val Pro Glu Ser
   1970 1975 1980
   Ser Ser Glu Thr Ser Ala Tyr Pro Glu Ala Gly Phe Gly Ala Ser Ala
   1985 1990 1995 2000
   Ala Pro Glu Ala Ser Arg Glu Asp Ser Gly Ser Pro Asp Leu Ser Glu
   2005 2010 2015
   Thr Thr Ser Ala Phe His Glu Ala Asn Leu Glu Arg Ser Ser Gly Leu
   2020 2025 2030
   Gly Val Ser Gly Ser Thr Leu Thr Phe Gln Glu Gly Glu Ala Ser Ala
   2035 2040 2045
   Ala Pro Glu Val Ser Gly Glu Ser Thr Thr Thr Ser Asp Val Gly Thr
   2050 2055 2060
   Glu Ala Pro Gly Leu Pro Ser Ala Thr Pro Thr Ala Ser Gly Asp Arg
   2065 2070 2075 2080
   Thr Glu Ile Ser Gly Asp Leu Ser Gly His Thr Ser Gln Leu Gly Val
   2085 2090 2095
   Val Ile Ser Thr Ser Ile Pro Glu Ser Glu Trp Thr Gln Gln Thr Gln
   2100 2105 2110
   Arg Pro Ala Glu Thr His Leu Glu Ile Glu Ser Ser Ser Leu Leu Tyr
   2115 2120 2125
   Ser Gly Glu Glu Thr His Thr Val Glu Thr Ala Thr Ser Pro Thr Asp
   2130 2135 2140
   Ala Ser Ile Pro Ala Ser Pro Glu Trp Lys Arg Glu Ser Glu Ser Thr
   2145 2150 2155 2160
   Ala Ala Ala Pro Ala Arg Ser Cys Ala Glu Glu Pro Cys Gly Ala Gly
   2165 2170 2175
   Thr Cys Lys Glu Thr Glu Gly His Val Ile Cys Leu Cys Pro Pro Gly
   2180 2185 2190
   Tyr Thr Gly Glu His Cys Asn Ile Asp Gln Glu Val Cys Glu Glu Gly
   2195 2200 2205
   Trp Asn Lys Tyr Gln Gly His Cys Tyr Arg His Phe Pro Asp Arg Glu
   2210 2215 2220
   Thr Trp Val Asp Ala Glu Arg Arg Cys Arg Glu Gln Gln Ser His Leu
   2225 2230 2235 2240
   Ser Ser Ile Val Thr Pro Glu Glu Gln Glu Phe Val Asn Asn Asn Ala
   2245 2250 2255
   Gln Asp Tyr Gln Trp Ile Gly Leu Asn Asp Arg Thr Ile Glu Gly Asp
   2260 2265 2270
   Phe Arg Trp Ser Asp Gly His Pro Met Gln Phe Glu Asn Trp Arg Pro
   2275 2280 2285
   Asn Gln Pro Asp Asn Phe Phe Ala Ala Gly Glu Asp Cys Val Val Met
   2290 2295 2300
   Ile Trp His Glu Lys Gly Glu Trp Asn Asp Val Pro Cys Asn Tyr His
   2305 2310 2315 2320
   Leu Pro Phe Thr Cys Lys Lys Gly Thr Val Ala Cys Gly Glu Pro Pro
   2325 2330 2335
   Val Val Glu His Ala Arg Thr Phe Gly Gln Lys Lys Asp Arg Tyr Glu
   2340 2345 2350
   Ile Asn Ser Leu Val Arg Tyr Gln Cys Thr Glu Gly Phe Val Gln Arg
   2355 2360 2365
   His Met Pro Thr Ile Arg Cys Gln Pro Ser Gly His Trp Glu Glu Pro
   2370 2375 2380
   Arg Ile Thr Cys Thr Asp Ala Thr Thr Tyr Lys Arg Arg Leu Gln Lys
   2385 2390 2395 2400
   Arg Ser Ser Arg His Pro Arg Arg Ser Arg Pro Ser Thr Ala His
   2405 2410 2415